# EUROPEAN PATENT SPECIFICATION

(11) **EP 1 635 803 B1**
(45) Date of publication and mention of the grant of the patent: **04.03.2009**
(21) Application number: 04734238.1
(22) Date of filing: 21.05.2004
(51) Int. Cl.: A61K 31/137, A61P 25/04, A61P 25/06

(54) **USE OF 2-AMINO-1-3-PROPANEDIOL DERIVATIVES FOR THE MANUFACTURE OF A MEDICAMENT FOR THE TREATMENT OF VARIOUS TYPES OF PAIN**
VERWENDUNG VON 2-AMINO-1,3-PROPANDIOLDERIVATEN ZUR HERSTELLUNG EINES MEDIKAMENTS ZUR BEHANDLUNG VON BESTIMMTEN SCHMERZEN
UTILISATION DE DERIVES DE 2-AMINO-1,3-PROPANEDIOL POUR LA PREPARATION D'UN MEDICAMENT DESTINE AU TRAITEMENT DE CERTAINES DOULEURS

(30) Priority: 06.06.2003 EP 03012864
(43) Date of publication of application: 22.03.2006
(73) Proprietor: Sanofi-Aventis Deutschland GmbH, 65929 Frankfurt am Main (DE)
(72) Inventor: MICHAELIS, Martin, 60325 Frankfurt (DE); GEISSLINGER, Gerd, 65812 Bad Soden (DE); SCHOLICH, Klaus, 63303 Dreieich (DE)
(86) International application number: PCT/EP2004/005470
(87) International publication number: WO 2004/110421

(56) References cited:
- EP-A- 0 627 406
- EP-A- 0 812 588
- EP-A- 1 002 792
- EP-A- 1 050 301
- WO-A-98/22100
- FR-A- 2 249 653
- KAHAN B D: "Sirolimus and FTY720: new approaches to transplant immunosuppression." TRANSPLANTATION PROCEEDINGS. UNITED STATES NOV 2002, vol. 34, no. 7, November 2002 (2002-11), pages 2520-2522, XP002258303 ISSN: 0041-1345
- SPIEGEL SARAH ET AL: "Sphingosine 1-phosphate, a key cell signaling molecule" JOURNAL OF BIOLOGICAL CHEMISTRY, vol. 277, no. 29, 19 July 2002 (2002-07-19), pages 25851-25854, XP002258383 ISSN: 0021-9258

## Description

The present invention relates to the use of compounds of the formula I-12, 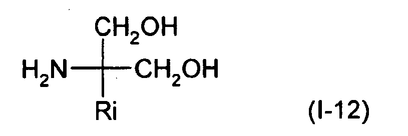 in which Ri has the meanings indicated below, for the preparation of pharmaceuticals for the prophylaxis or treatment of various types of pain.

The use of 2-amino-2-(2-(4-octylphenyl)propane-1,3-diol and of other 2-aminopropane-1,3-diol derivatives for the treatment of autoimmune diseases, transplant rejections and allergic conditions are disclosed in EP 1050301, EP 0812588, EP 1002792, WO 98/22100 and in Kahan B.D. (Sirulimus and FTY720: new approaches to transplant immunosuppression; Transplantation Proceedings; US; Nov. 2002; Vol. 34; No. 7, pages 2520-2522; XP 002258303). Compounds of the formula I-12 are described in EP 0 627 406 as medicaments for immunosuppression, suppressing rejection, autoimmune diseases, rheumatoid arthritis, psoriasis, atopic dermatitis, bronchial asthma, pollinosis or Behcet's disease.

Pain is a complex subjective sensation reflecting real or potential tissue damage and the affective response to it. Acute pain is a physiological signal indicating a potential or actual injury. Chronic pain can either be somatogenetic (organic) or psychogenic. Chronic pain is frequently accompanied or followed by vegetative signs, which often ' result in depression.

Somatogenetic pain may be of nociceptive origin, inflammatory or neuropathic. Nociceptive pain is judged to be commensurate with ongoing activation of somatic or visceral pain-sensitive nerve fibers. Neuropathic pain results from dysfunction in the nervous system; it is believed to be sustained by aberrant somatosensory processes in the peripheral nervous system, the central nervous system (CNS), or both. (For an overview of pain mechanisms, see for example Scholz and Woolf, 2002; Julius and Basbaum, 2001, Woolf and Mannion, 1999; Wood, J.D., 2000; Woolf and Salter, 2000.)

Chronic pain results in individual suffering and social economic costs of tremendous extent. Existing pharmacological pain therapies are widely unsatisfying both in terms of efficacy and of safety.

Up to now, two classes of analgesics are mainly employed for the treatment of pain:
Non-opioid analgesics, mostly acetaminophen and NSAIDS (non-steroidal antiinflammatory drugs) and opioid (narcotic) agonists (wherein "opioid" is a generic term for natural or synthetic substances that bind to specific opioid receptors in the CNS, producing an agonist action). Unfortunately both analgesic classes, opioids and non-opioids, have several unwanted side effects. The most serious side effects of opioids are the possibility of inhibition of the respiratory system and after long-term treatment the possibility of addiction (Schaible H.G., Vanegas H.: How do we manage chronic pain? Baillieres Best. Pract. Res. Clin. Rheumatol. 2000 Dec;14(4):797-811). NSAIDs, a major class of non-opioids, on the other hand, can induce a variety of gastrointestinal complications such as ulcers and bleeding, but also kidney damage (Schaible H.G., Vanegas H., 2000). It has been estimated that in the U.S.A. about 16.000 patients die every year because of severe gastro-intestinal complications caused by conventional NSAIDs.

In light of the severe drawbacks connected with state of the art pain treatments, there is a great need for novel classes of pain modulating drugs. Especially in light of the vast gap between the fast advancing understanding of the neurobiology of pain and the unmet clinical need to provide effective treatments without the drawbacks of state of the art treatments, efforts need to be directed to the discovery of new targets for novel classes of analgesics.

It was found that sphingosine-1-phosphate (S1P) is involved in nociceptive processing and is able to decrease pain. S1P is able to interact with at least one S1P receptor, activates the receptor and lowers intracellular cyclicadenosine mono phosphate (AMP). S1 P can bind to a family of five G-Protein-coupled receptors S1P₁-₅ also known as Endothelial Differentiation Gene receptor EDG 1,3,5,6 and 8.

Members of the S1 P-receptor family regulate functions involved in neural cell morphology, tumor cell invasiveness, cell proliferation, angiogenesis, vascular maturation, and inhibition of neutrophil motility and migration (Kluk M. J. and Hla T. (2002); Biochim Biophys Acta 1582, 72-80; Spiegel S. and Milstien S. (2002), J Biol Chem 277, 25851-25854). It is well known that several of these actions are mediated at least in part by the inhibition of cAMP synthesis. S1 P achieves its adenylate cyclase (AC) inhibitory actions by activating PAM and the inhibitory G-protein Gᵢ. S1 P treatment results in a change of the cellular localization of PAM and inhibition of AC enzyme activity.
The compounds of the formulae I-12, I-13 and S1P are S1 P-receptor agonists. Therefore, the compounds of formulae I-12 and I-13 can be used for treatment of pain.

The present invention satisfies the above needs by providing compounds of the formulae I-12 and I-13, which can exhibit pain inhibitory activity and offer fewer side effects.

Thus, the present invention relates to the use of a 2-amino-1,3-propanediol compound of the formula I-12, 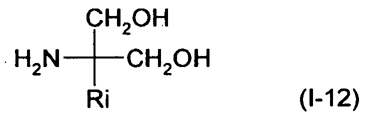 for the preparation of pharmaceuticals for the prophylaxis or treatment of chronic or acute pain, wherein
- Ri: is a phenylalkyl, wherein the alkyl moiety is a straight- or branched chain alkyl having 1 to 30 carbon atoms, said phenylalkyl being substituted by a straight- or branched chain C6-C14 alkyl optionally substituted by halogen, a straight- or branched chain C6-C14 alkoxy optionally substituted by halogen or a straight- or branched chain C6-C14 alkenyloxy,
wherein the alkyl moiety of phenylalkyl may be substituted by hydroxyl, or a pharmaceutically acceptable salt thereof.

The invention also refers to the use of a 2-amino-1,3-propanediol compound of the formula I-13, 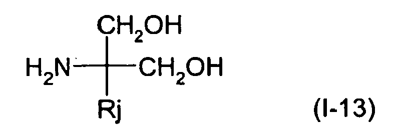 for the preparation of pharmaceuticals for the prophylaxis or treatment of chronic or acute pain, wherein
- Rj: is a phenylalkyl, wherein the alkyl moiety is a C2-C6 alkyl optionally substituted by hydroxy, said phenylalkyl being substituted by a straight- or branched chain C6-C14 alkyl optionally substituted by halogen, a straight- or branched chain C6-C14 alkoxy optionally substituted by halogen, or a straight- or branched chain C6-C14 alkenyloxy, or a pharmaceutically acceptable salt thereof.

The present invention also relates to the use of a 2-amino-1,3-propanediol compound, which is selected from:
2-amino-2-[2-(4-heptylphenyl)ethyl]-1,3-propanediol,
2-amino-2-[2-(4-octylphenyl)ethyl]-1,3-propanediol,
2-amino-2-[2-(4-octylphenyl)ethyl]-1,3-propanediol hydrochloride,
2-amino-2-[2-(4-nonylphenyl)ethyl]-1,3-propanediol,
2-amino-2-[2-(4-decylphenyl)ethyl]-1,3-propanediol,
2-amino-2-[2-(4-undecylphenyl)ethyl]-1,3-propanediol,
2-amino-2-[2-(4-dodecylphenyl)ethyl]-1,3-propanediol,
2-amino-2-[2-(4-tridecylphenyl)ethyl]-1,3-propanediol,
2-amino-2-[2-(4-tetradecylphenyl)ethyl]-1,3-propanediol,
2-amino-2-[2-(4-hexyloxyphenyl)ethyl]-1,3-propanedial,
2-amino-2-[2-(4-heptyloxyphenyl)ethyl]-1,3-propanediol,
2-amino-2-[2-(4-octyloxyphenyl)ethyl]-1,3-propanediol,
2-amino-2-[2-(4-nonyloxyphenyl)ethyl]-1,3-propanediol,
2-amino-2-[2-(4-decyloxyphenyl)ethyl]-1,3-propanediol,
2-amino-2-[2-(4-undecyloxyphenyl)ethyl]-1,3-propanediol,
2-amino-2-[2-(4-dodexyloxyphenyl)ethyl]-1,3-propanediol,
2-amino-2-[2-(4-tridecyloxyphenyl)ethyl]-1,3-propanediol,
2-amino-2-[2-(4-(8-fluorooctyl)phenyl)ethyl]-1,3-propanediol,
2-amino-2-[2-(4-(12-fluorododecyl)phenyl)ethyl]-1,3-propanediol,
2-amino-2-[2-(4-(7-fluoroheptyloxy)phenyl)ethyl]-1,3-propanediol,
2-amino-2-[2-(4-(11-fluoroundecyloxy)phenyl)ethyl]-1,3-propanediol and
2-amino-2-[2-(4-(7-octenyloxy)phenyl)ethyl]-1,3-propanediol, or a pharmaceutically acceptable salt thereof.

As used herein, the term "alkyl having 1 to 30 carbon atoms" is to be understood in the broadest sense to mean hydrocarbon residues which can be linear, i.e. straight-chain, or branched and which can be an acyclic or cyclic residues or comprise any combination of an acyclic and cyclic subunits. Further, the term alkyl as used herein expressly includes saturated groups as well as unsaturated groups which latter groups contain one or more, for example one, two, three or four double bonds and/or triple bonds, provided that the double bonds are not located within a cyclic alkyl group in such a manner that an aromatic system results. All these statements also apply if an alkyl group occurs as a substituent on another residue, for example in an alkyloxy residue, an alkyloxycarbonyl residue or an arylalkyl residue. Examples of alkyl having 1 to 30 carbon atoms or alkylene having 1 to 30 carbon atoms are alkyl residues such as methyl, methylene, ethyl, ethylene, propyl, propylene, butyl, butylene, pentyl, pentylene, hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl. The n-isomers of all these residues, isopropyl, isobutyl, 1-methylbutyl, isopentyl, neopentyl, 2,2-dimethylbutyl, 2-methylpentyl, 3-methylpentyl, isohexyl, sec-butyl, tBu, tert-pentyl, sec-butyl, tert-butyl or tert-pentyl.

The term "alkenyl having 2 to 30 carbon atoms" is an unsaturated alkyl residue having 2 to 30 carbon atoms and contains 1, 2, 3 or 4 double bonds and can be derived from alkyl as defined above such as vinyl, 1-propenyl, 2-propenyl (= allyl), 2-butenyl, 3-butenyl, 2-methyl-2-butenyl, 3-methyl-2-butenyl, 5-hexenyl or 1,3-pentadienyl.

Unless stated otherwise, the term alkyl preferably comprises acyclic saturated hydrocarbon residues which have from one to six carbon atoms and which can be linear or branched. A particular group of saturated acyclic alkyl residues is formed by (C₁-C₄)-alkyl residues like methyl, ethyl, n-propyl, isopropyl, n-butyl, isobutyl, sec-butyl and tBu.

The term "phenylalkyl" is understood as meaning an aryl residue, which is substituted by alkyl having 1 to 30 carbon atoms. Examples of phenylalykl are benzyl, phenylethyl or phenylpropyl.

The term "alkoxy" is understood as meaning a straight or branched chain alkoxy having 1 to 20 carbon atoms, such as methoxy, ethoxy, propoxy, butoxy, isobutoxy, pentyloxy, hexyloxy, heptyloxy or octyloxy.

Halogen is fluorine, chlorine, bromine or iodine, preferably fluorine, chlorine or bromine, particularly preferably chlorine or bromine.

The term "pain" describes acute and chronic pain states.
Examples for chronic pain states are:
Chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e.g. after amputation, trigeminal neuralgia, migraine or post herpetic neuralgia.
Examples for acute pain are:
Pain after injuries, Postoperative pain, Pain during acute gout, Pain during operations, such as jaw surgery.

Optically active carbon atoms present in the compounds of the formulae I-12 or I-13 can independently of each other have R configuration or S configuration. The compounds of the formulae I-12 or I-13 can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of the formulae I-12 or I-13, and it comprises all ratios of the stereoisomers in the mixtures. In case the compounds of the formulae I-12 or I-13 can be present as E isomers or Z isomers (or cis isomers or trans isomers) the invention relates both to pure E isomers and pure Z isomers and to E/Z mixtures in all ratios. The invention also comprises all tautomeric forms of the compounds of the formulae I-12 or I-13.

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds of the formulae I-12 or I-13 can also be obtained by employing stereochemically uniform starting materials or by using stereoselective reactions.

Physiologically tolerable salts of the compounds of formulae I-12 or I-13 are nontoxic salts that are physiologically acceptable, in particular pharmaceutically utilizable salts. Such salts of compounds of the formulae I-12 or I-13 containing acidic groups, for example a carboxyl group COOH, are for example alkali metal salts or alkaline earth metal salts such as sodium salts, potassium salts, magnesium salts and calcium salts, and also salts with physiologically tolerable quaternary ammonium ions such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in thee compounds of the formulae I-12 or I-13, for example carbamoyl groups or guanidino groups, form acid addition salts, for example with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluehesulfonic acid or amino acids such as lysine salts. Compounds of the formulae I-12 or I-13, which simultaneously contain a basic group and an acidic group, for example a guanidino group and a carboxyl group, can also be present as zwitterions (betaines), which are likewise included in the present invention.

Salts of compounds of the formulae I-12 or I-13 can be obtained by customary methods known to those skilled in the art, for example by combining a compound of the formulae I-12 or I-13 with an inorganic or organic acid or base in a solvent or dispersant, or from other salts by cation exchange or anion exchange. The present invention also includes all salts of the compounds of the formulae I-12 or I-13 which, because of low physiologically tolerability, are not directly suitable for use in pharmaceuticals but are suitable, for example, as intermediates for carrying out further chemical modifications of the compounds of the formulae I-12 or I-13 or as starting materials for the preparation of physiologically tolerable salts.
The present invention furthermore includes all solvates of compounds of the formulae I-12 or I-13, for example hydrates or adducts with alcohols.

In general compounds of the formulae I-12 or I-13 can be prepared as described in EP 0 627 406.

The compounds of the present invention are S1 P-receptor agonists and thus the compounds of the formulae I-12 or I-13 can be used for decreasing pain.

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays or transdermal patches.

The pharmaceutical preparations according to the invention are prepared in a manner known per se and familiar to one skilled in the art, pharmaceutically acceptable inert inorganic and/or organic carriers being used in addition to the compound(s) of the formulae I-12 or I-13 and/or its (their) physiologically tolerable salts. For the production of pills, tablets, coated tablets and hard gelatin capsules it is possible to use, for example, lactose, cornstarch or derivatives thereof, talc, stearic acid or its salts, etc. Carriers for soft gelatin capsules and suppositories are, for example, fats, waxes, semisolid and liquid polyols, natural or hardened oils, etc. Suitable carriers for the production of solutions, for example injection solutions, or of emulsions or syrups are, for example, water, saline, alcohols, glycerol, polyols, sucrose, invert sugar, glucose, vegetable oils, etc. Suitable carriers for microcapsules, implants or rods are, for example, copolymers of glycolic acid and lactic acid. The pharmaceutical preparations normally contain about 0.5 % to 90 % by weight of the compounds of the formulae I-12 or I-13 and/or their physiologically tolerable salts. The amount of the active ingredient of the formulae I-12 or I-13 and/or its physiologically tolerable salts in the pharmaceutical preparations normally is from about 0.5 mg to about 1000 mg, preferably from about 1 mg to about 500 mg.

In addition to the active ingredients of the formulae I-12 or I-13 and/or their physiologically acceptable salts and to carrier substances, the pharmaceutical preparations can contain additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formulae I-12 or I-13 and/or their physiologically tolerable salts. In case a pharmaceutical preparation contains two or more compounds of the formulae I-12 or I-13 the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the compounds of the formulae I-12 or I-13 allows a great deal of control over the biological and physico-chemical properties of the compounds and thus allows the selection of such desired compounds. Furthermore, in addition to at least one compound of the formulae I-12 or I-13 and/or its physiologically tolerable salts, the pharmaceutical preparations can also contain one or more other therapeutically or prophylactic ally active ingredients.

### Examples

### Example 1: Determination of the analgesic effect of S1P

The analgesic effect of sphingosine-1-phosphate (S1P) was determined by intrathecal application to the spinal cord by.
a) Implantation of lumbar intrathecal catheters:
   Wild type Sprague Dawley rats were purchased from Charles River Wiga GmbH (Sulzfeld, Germany). The animals had free access to food and water prior to the experiments. They were maintained in climate- and light-controlled rooms (24 + 0.5°C). Each animal was used at one occasion only. In all experiments the ethics guidelines for investigations in conscious animals were obeyed, and the procedures were approved by the local Ethics Committee.
   Rats were anesthetized with ketamine (60 mg/kg i.p.) and midazolam (0.5 to 1 mg/kg i.p.). The skin was incised above the vertebral column from vertebrae Th13 up to L3. Muscle tissue around L2-3 was cleared away. The processus spinosus of L3 was removed and a laminectomy was done at L2. Polyethylene catheters (ID 0.28 mm, OD 0.61 mm) were then inserted into the peridural space so that the tip of the catheter reached Th9-10. The catheter was fixed with cyanacrylate glue and was externalized in the neck region and the skin was sutured.
b) Infusion of PAM oligonuleotides.
   Three days after surgery rats were placed into a "freely moving system" (CMA, Stockholm, Sweden) 20 µl of 10 µM S1P, purchased from Tocris (Ellisville, MO), were infused through the catheter.
c) Formalin test:
   Within 15 min after stopping the infusion the formalin test was performed. 50 µl of a 5% formaldehyde solution were injected subcutaneously (s: c.) into the dorsal surface of one hind paw. Flinches were counted in one-minute intervals up to 60 min starting right after formalin injection. Flinches of 5 min intervals were summarized as mean flinches per minute. To compare the nociceptive behavior between groups the sum of flinches during the one-hour observation period were submitted to the Students t-test.
   At the end of the formalin test, the rats were killed.
d) Results:
   S1P was dissolved in DMSO to a final concentration of 2.5 mM and then diluted 1:250 in PBS. 20µl of 10µM S1P or 20µl 0.1 M phosphate, buffered saline, pH 7.2 (PBS,) in DMSO were given to adult rats by intrathecal application 15 minutes prior to the formalin injection. Then, flinches were counted in 5 minutes intervals over a period of 60 minutes.
   A significant decrease in the number of nociceptive responses for phase 2A, which is the time from 20 to 35 minutes after formalin injection, could be detected as compared to PBS/DMSO-treated animals. These experiments clearly demonstrated that exogenous S1P acts as an analgesic.
   Table 1 shows the results of the mean of six animal experiments + SEM.

**Table 1:**

| Time | Mean | SEM | Mean | SEM |
|---|---|---|---|---|
| (min) | control | control | S1P 10µM | S1P 10 µM |
| 5 | 68.0 | 11.4 | 60.9 | 9.6 |
| 10 | 29.3 | 4.1 | 24.6 | 5.7 |
| 15 | 23.8 | 5.3 | 12.7 | 3.0 |
| 20 | 41.5 | 6.5 | 17.1 | 3.4 |
| 25 | 54.1 | 12.0 | 19.6 | 5.1 |
| 30 | 68.3 | 10.4 | 24.7 | 5.5 |
| 35 | 74.7 | 12.5 | 31.4 | 5.5 |
| 40 | 58.0 | 4.0 | 41.9 | 6.6 |
| 45 | 64.9 | 6.0 | 49.3 | 9.8 |
| 50 | 60.7 | 9.0 | 41.3 | 9.6 |
| 55 | 58.5 | 5.2 | 49.0 | 9.7 |
| 60 | 53.7 | 12.9 | 38.0 | 7.9 |

### Example 2: Determination of the analgesic effect of 2-amino-2-[2-(4-octylphenyl)ethyl]-1,3-propanediol hydrochloride

Animals: Male Sprague Dawley rats weighing 300-350 g were purchased from Charles River Wiga GmbH (Sulzfeld, Germany). The animals had free access to food and water prior to the experiments. They were maintained in climate- and light-controlled rooms (24 + 0.5°C). Each animal was used at one occasion only. In all experiments the ethics guidelines for investigations in conscious animals were obeyed and the procedures were approved by the local Ethics Committee.
Formalin test: The formalin assay was performed in a dedicated room with restriction on sound level and activity. 2-amino-2-[2-(4-octylphenyl)ethyl]-1,3-propanediol hydrochloride (compound 1) was dissolved in polyethylene-glycol (PEG) at a concentration of 1 mg/ml and injected interperitoneally (1 mg/kg) 3 hours prior to the formalin tests were performed with compound 1. 50 µl of a 5% formaldehyde solution was injected subcutaneously (s.c.) into the dorsal surface of one hind paw. Rats were placed into a plexiglas chamber surrounded by mirrors to allow an unobstructed view of the paws. Flinches were counted in one-minute intervals up to 60 min starting right after formalin injection. Flinches of 5 min intervals were summarized as mean flinches per minute + SEM. Statistical analysis was performed using the student's t-test (* p < 0.04).
A significant decrease in the number of nociceptive responses for phase 2A, which is the time from 20 to 35 minutes after formalin injection, could be detected as compared to the control animals (four animals). These experiments clearly demonstrated that compound 1 is an analgesic.

Table 2 shows the results of the mean of five animal experiments + SEM.

**Table 2:**

| Time | Compound 1 | SEM | | SEM |
|---|---|---|---|---|
| (min) | (1 mg/kg) | (compound 1) | Control | (control) |
| 5 | 85.4 | 13.2 | 95.0 | 3.4 |
| 10 | 41.2 | 4.9 | 29.0 | 6.7 |
| 15 | 33.6 | 6.3 | 40.8 | 1.7 |
| 20 | 29.6 | 7.2 | 41.0 | 5.4 |
| 25 | 31.6 | 4.1 | 61.5 | 4.5 |
| 30 | 51.4 | 13.1 | 72.0 | 9.0 |
| 35 | 49.2 | 4.8 | 69.0 | 6.4 |
| 40 | 48.0 | 5.8 | 76.3 | 5.6 |
| 45 | 48.6 | 10.7 | 67.5 | 7.9 |
| 50 | 39.8 | 4.6 | 45.5 | 5.5 |
| 55 | 42.6 | 13.3 | 38.5 | 5.9 |
| 60 | 42.2 | 5.0 | 37.0 | 4.4 |

| | | | | |
|---|---|---|---|---|
| Compound 1 n=5 rats control n=4 rats | | | | |

### Example 3: Determination of the analgesic or antinociceptive effect of a S1P receptor agonist

The analgesic or antinociceptive effect of a compound of the formulae I-12 or I-13 can be determined in the formalin model of acute pain as described in example 1. The effect of a compound of the formulae I-12 or I-13 can be determined either by inthathecal, intravenous, subcutaneous, interperitoneal, topical or oral application and consecutive testing of its analgesic or antinociceptive effect by means of the flinch test. This approach allows the molecule to enter the tissue and mimic the actions of physiological S1P towards adenylate cyclase.

## Claims

1. The use of a 2-amino-1,3-propanediol compound of the formula I-12, 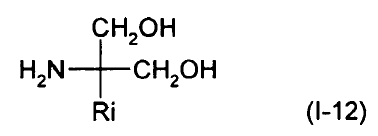 for the preparation of pharmaceuticals for the prophylaxis or treatment of chronic or acute pain, wherein
Ri is a phenylalkyl, wherein the alkyl moiety is a straight- or branched chain alkyl having 1 to 30 carbon atoms, said phenylalkyl being substituted by a straight- or branched chain C6-C14 alkyl optionally substituted by halogen, a straight- or branched chain C6-C14 alkoxy optionally substituted by halogen or a straight- or branched chain C6-C14 alkenyloxy,
wherein the alkyl moiety of phenylalkyl may be substituted by hydroxyl, or a pharmaceutically acceptable salt thereof.

2. The use of a 2-amino-1,3-propanediol compound of the formula I-13 as claimed in claim 1, 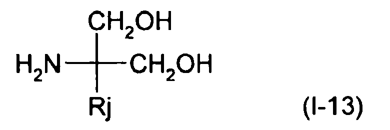 for the preparation of pharmaceuticals for the prophylaxis or treatment of chronic or acute pain, wherein
Rj is a phenylalkyl, wherein the alkyl moiety is a C2-C6 alkyl optionally substituted by hydroxyl, said phenylalkyl being substituted by a straight-or branched chain C6-C14 alkyl optionally substituted by halogen, a straight- or branched chain
C6-C14 alkoxy optionally substituted by halogen, or a straight- or branched chain C6-C14 alkenyloxy, or a pharmaceutically acceptable salt thereof.

3. The use as claimed in claim 1 of a 2-amino-1,3-propanediol compound, which is selected from:
2-amino-2-[2-(4-heptylphenyl)ethyl]-1,3-propanediol,
2-amino-2-[2-(4-octylphenyl)ethyl]-1,3-propanediol,
2-amino-2-[2-(4-octylphenyl)ethyl]-1,3-propanediol hydrochloride,
2-amino-2-[2-(4-nonylphenyl)ethyl]-1,3-propanediol,
2-amino-2-[2-(4-decylphenyl)ethyl]-1,3-propanediol,
2-amino-2-[2-(4-undecylphenyl)ethyl]-1, 3-propanediol,
2-amino-2-[2-(4-dodecylphenyl)ethyl]-1,3-propanediol,
2-amino-2-[2-(4-tridecylphenyl)ethyl]-1,3-propanediol,
2-amino-2-[2-(4-tetradecylphenyl)ethyl]-1,3-propanediol,
2-amino-2-[2-(4-hexyloxyphenyl)ethyl]-1,3-propanediol,
2-amino-2-[2-(4-heptyloxyphenyl)ethyl]-1,3-propanediol,
2-amino-2-[2-(4-octyloxyphenyl)ethyl]-1,3-propanediol,
2-amino-2-[2-(4-nonyloxyphenyl)ethyl]-1,3-propanediol,
2-amino-2-[2-(4-decyloxyphenyl)ethyl]-1,3-propanediol,
2-amino-2-[2-(4-undecyloxyphenyl)ethyl]-1,3-propanediol,
2-amino-2-[2-(4-dodexyloxyphenyl)ethyl]-1,3-propanediol,
2-amino-2-[2-(4-tridecyloxyphenyl)ethyl]-1, 3-propanediol,
2-amino-2-[2-(4-(8-fluorooctyl)phenyl)ethyl]-1,3-propanediol,
2-amino-2-[2-(4-(12-fluorododecyl)phenyl)ethyl]-1,3-propanediol,
2-amino-2-[2-(4-(7-fluoroheptyloxy)phenyl)ethyl]-1,3-propanediol,
2-amino-2-[2-(4-(11-fluoroundecyloxy)phenyl)ethyl]-1,3-propanediol and
2-amino-2-[2-(4-(7-octenyloxy)phenyl)ethyl]-1,3-propanediol, or a pharmaceutically acceptable salt thereof.

4. The use of a 2-amino-1,3-propanediol compound as claimed in one or more of claims 1 to 3 for the preparation of pharmaceuticals for the prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e.g. after amputation, trigeminal neuralgia, migraine or post herpetic neuralgia.

5. The use of a 2-amino-1,3-propanediol compound as claimed in one or more of claims 1 to 3 for the preparation of pharmaceuticals for the prophylaxis or treatment of acute pain, wherein acute pain is selected from pain after injuries, postoperative pain, pain during acute gout, pain during operations, such as jaw surgery.

## Patentansprüche

1. Verwendung einer 2-Amino-1,3-propandiolverbindung der Formel I-12 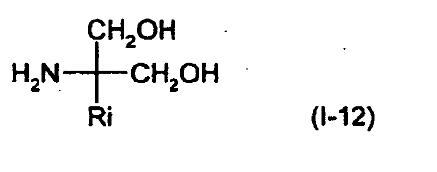 zur Herstellung von Pharmazeutika für die Prophylaxe oder Behandlung von chronischen oder akuten Schmerzen,
wobei
Ri für Phenylalkyl steht, wobei es sich bei der Alkylgruppe um geradkettiges oder verzweigtes Alkyl mit 1 bis 30 Kohlenstoffatomen handelt, wobei der Phenylalkylrest durch eine gegebenenfalls halogensubstituierte geradkettige oder verzweigte C6-C14-Alkylgruppe, eine gegebenenfalls halogensubstituierte geradkettige oder verzweigte C6-C14-Alkoxygruppe oder eine geradkettige oder verzweigte C6-C14-Alkenyloxygruppe substituiert ist,
wobei die Alkylgruppe des Phenylalkylrests durch Hydroxy substituiert sein kann, oder eines pharmazeutisch unbedenklichen Salzes davon.

2. Verwendung einer 2-Amino-1,3-propandiolverbindung der Formel I-13 nach Anspruch 1 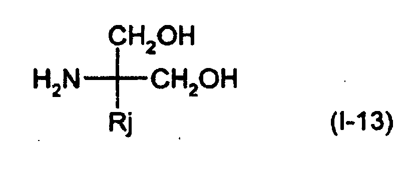 zur Herstellung von Pharmazeutika zur Prophylaxe oder Behandlung von chronischen oder akuten Schmerzen, wobei
Rj für Phenylalkyl steht, wobei es sich bei der Alkylgruppe um gegebenenfalls hydroxysubstituiertes C2-C6-Alkyl handelt, wobei der Phenylalkylrest durch gegebenenfalls halogensubstituiertes geradkettiges oder verzweigtes C6-C14-Alkyl, gegebenenfalls halogensubstituiertes geradkettiges oder verzweigtes C6-C14-Alkoxy oder geradkettiges oder verzweigtes C6-C14-Alkenyloxy substituiert ist, oder eines pharmazeutisch unbedenklichen Salzes davon.

3. Verwendung nach Anspruch 1 einer 2-Amino-1,3-propandiolverbindung, ausgewählt aus:
2-Amino-2-[2-(4-heptylphenyl)ethyl]-1,3-propandiol,
2-Amino-2-[2-(4-octylphenyl)ethyl]-1,3-propandiol,
2-Amino-2-[2-(4-octylphenyl)ethyl]-1,3-propandiolhydrochlorid,
2-Amino-2-[2-(4-nonylphenyl)ethyl]-1,3-propandiol,
2-Amino-2-[2-(4-decylphenyl)ethyl]-1,3-propandiol,
2-Amino-2-[2-(4-undecylphenyl)ethyl]-1,3-propandiol,
2-Amino-2-[2-(4-dodecylphenyl)ethyl]-1,3-propandiol,
2-Amino-2-[2-(4-Tridecylphenyl)ethyl]-1,3-propandiol,
2-Amino-2-[2-(4-tetradecylphenyl)ethyl]-1,3-propandiol,
2-Amino-2-[2-(4-hexyloxyphenyl)ethyl]-1,3-propandiol,
2-Amino-2-[2-(4-heptyloxyphenyl)ethyl]-1,3-propandiol,
2-Amino-2-[2-(4-octyloxyphenyl)ethyl]-1,3-propandiol,
2-Amino-2-[2-(4-nonyloxyphenyl)ethyl]-1,3-propandiol,
2-Amino-2-[2-(4-decyloxyphenyl)ethyl]-1,3-propandiol,
2-Amino-2-[2-(4-undecyloxyphenyl)ethyl]-1,3-propandiol,
2-Amino-2-[2-(4-dodecyloxyphenyl)ethyl]-1,3-propandiol,
2-Amino-2-[2-(4-tridecyloxyphenyl)ethyl]-1,3-propandiol,
2-Amino-2-[2-(4-(8-fluoroctyl)phenyl)ethyl]-1,3-propandiol,
2-Amino-2-[2-(4-(12-fluordodecyl)phenyl)ethyl]-1,3-propandiol,
2-Amino-2-[2-(4-(7-fluorheptyloxy)phenyl)ethyl]-1,3-propandiol,
2-Amino-2-[2-(4-(11-fluorundecyloxy)phenyl)ethyl]-1,3-propandiol und
2-Amino-2-[2-(4-(7-octenyloxy)phenyl)ethyl]-1,3-propandiol, oder ein pharmazeutisch unbedenkliches Salz davon.

4. Verwendung einer 2-Amino-1,3-propandiolverbindung nach einem oder mehreren der Ansprüche 1 bis 3 zur Herstellung von Pharmazeutika zur Prophylaxe oder Behandlung von chronischen Schmerzen, wobei die chronischen Schmerzen ausgewählt sind aus chronischen Muskelkrankheiten wie Rückenschmerzen, Menstruationsschmerzen, mit Osteoarthritis verbundene Schmerzen, mit rheumatoider Arthritis verbundene Schmerzen, mit gastrointestinalen Entzündungen verbundene Schmerzen, mit Entzündungen des Herzmuskels verbundene Schmerzen, mit Multipler Sklerose verbundene Schmerzen, mit Neuritis verbundene Schmerzen, mit AIDS verbundene Schmerzen, mit Chemotherapie verbundene Schmerzen, Tumorschmerzen, neuropathische Schmerzen, z.B. nach einer Amputation, trigeminale Neuralgie, Migräne oder postherpetische Neuralgie.

5. Verwendung einer 2-Amino-1,3-propandiolverbindung nach einem oder mehreren der Ansprüche 1 bis 3 zur Herstellung von Pharmazeutika zur Prophylaxe oder Behandlung von akuten Schmerzen, wobei die akuten Schmerzen ausgewählt sind aus Schmerzen nach Verletzungen, postoperativen Schmerzen, mit akuter Gicht verbundene Schmerzen und Schmerzen während Operationen wie z.B. Kieferchirurgie.

## Revendications

1. Emploi d'un 2-amino-1,3-propanediol de formule I-12, 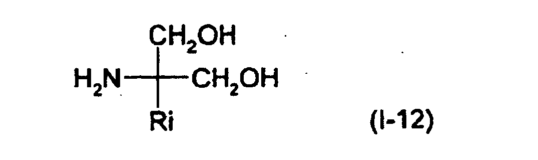 dans l'élaboration de produits pharmaceutiques pour le traitement prophylactique ou thérapeutique de la douleur chronique ou aiguë, où
Ri représente un groupement phénylalkyle, où la partie alkyle représente une chaîne alkyle linéaire ou ramifiée comportant entre 1 et 30 atomes de carbone, ledit phénylalkyle étant substitué par une chaîne alkyle en C6-C14 linéaire ou ramifiée éventuellement substituée par un atome d'halogène, une chaîne alkoxy en C6-C14 linéaire ou ramifiée éventuellement substituée par un atome d'halogène ou une chaîne alcényloxy en C6-C14 linéaire ou ramifiée,
où la partie alkyle du groupement phénylalkyle peut être substituée par un groupement hydroxyle, ou d'un sel de qualité pharmaceutique dudit composé.

2. Emploi d'un 2-amino-1,3-propanediol de formule I-13 selon la revendication 1, 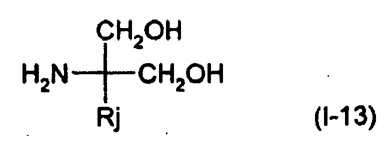 dans l'élaboration de produits pharmaceutiques pour le traitement prophylactique ou thérapeutique de la douleur chronique ou aiguë, où
Rj représente un groupement phénylalkyle, où la partie alkyle représente un groupement alkyle en C2-C6 éventuellement substitué par un groupement hydroxyle, ledit phénylalkyle étant substitué par une chaîne alkyle en C6-C14 linéaire ou ramifiée éventuellement substituée par un atome d'halogène, une chaîne alkoxy en C6-C14 linéaire ou ramifiée éventuellement substituée par un atome d'halogène ou une chaîne alcényloxy en C6-C14 linéaire ou ramifiée, ou d'un sel de qualité pharmaceutique dudit composé.

3. Emploi selon la revendication 1 d'un 2-amino-1,3-propanediol, sélectionné parmi les composés suivants :
2-amino-2-[2-(4-heptylphényl)éthyl]-1,3-propanediol,
2-amino-2-[2-(4-octylphényl)éthyl]-1,3-propanediol,
chlorhydrate de 2-amino-2-[2-(4-octylphényl)éthyl]-1,3-propanediol,
2-amino-2-[2-(4-nonylphényl)éthyl]-1,3-propanediol,
2-amino-2-[2-(4-décylphényl)éthyl]-1,3-propanediol,
2-amino-2-[2-(4-undécylphényl)éthyl]-1,3-propanediol,
2-amino-2-[2-(4-dodécylphényl)éthyl]-1,3-propanediol,
2-amino-2-[2-(4-tridécylphényl)éthyl]-1,3-propanediol,
2-amino-2-[2-(4-tétradécylphényl)éthyl]-1,3-propanediol,
2-amino-2-[2-(4-hexyloxyphényl)éthyl]-1,3-propanediol,
2-amino-2-[2-(4-heptyloxyphényl)éthyl]-1,3-propanediol,
2-amino-2-[2-(4-octyloxyphényl)éthyl]-1,3-propanediol,
2-amino-2-[2-(4-nonyloxyphényl)éthyl]-1,3-propanediol,
2-amino-2-[2-(4-décyloxyphényl)éthyl]-1,3-propanediol,
2-amino-2-[2-(4-undécyloxyphényl)éthyl]-1,3-propanediol,
2-amino-2-[2-(4-dodéxyloxyphényl)éthyl]-1,3-propanediol,
2-amino-2-[2-(4-tridécyloxyphényl)éthyl]-1,3-propanediol,
2-amino-2-[2-(4-(8-fluorooctyl)phényl)éthyl]-1,3-propanediol,
2-amino-2-[2-(4-(12-fluorododécyl)phényl)éthyl]-1,3-propanediol,
2-amino-2-[2-(4-(7-fluoroheptyloxy)phényl)éthyl]-1,3-propanediol,
2-amino-2-[2-(4-(11-fluoroundécyloxy)phényl)éthyl]-1,3-propanediol et
2-amino-2-[2-(4-(7-octényloxy)phényl)éthyl]-1,3-propanediol,
ou d'un sel de qualité pharmaceutique dudit composé.

4. Emploi d'un 2-amino-1,3-propanediol selon une ou plusieurs des revendications 1 à 3 dans l'élaboration de produits pharmaceutiques pour le traitement prophylactique ou thérapeutique de la douleur chronique, la douleur chronique étant sélectionnée parmi les maladies musculaires chroniques comme le mal de dos, les règles douloureuses, la douleur pendant l'arthrose, la douleur pendant la polyarthrite rhumatoïde, la douleur pendant une inflammation gastro-intestinale, la douleur pendant une myocardite, la douleur pendant une sclérose en plaques, la douleur pendant une névrite, la douleur pendant le SIDA, la douleur pendant une chimiothérapie, les douleurs tumorales, la douleur névropathique, par exemple après une amputation, la névralgie trigéminale, la migraine ou la névralgie post-herpétique.

5. Emploi d'un 2-amino-1,3-propanediol selon une ou plusieurs des revendications 1 à 3 dans l'élaboration de produits pharmaceutiques pour le traitement prophylactique ou thérapeutique de la douleur aiguë, où la douleur aiguë est sélectionnée parmi la douleur résultant de blessures, la douleur postopératoire, la douleur pendant la goutte aiguë, la douleur pendant des opérations, par exemple une chirurgie maxillaire.
